**Europäisches Patentamt**

(19) **European Patent Office**

**Office européen des brevets**

(11) Numéro de publication: **0 136 297**
**B1**

(12) # FASCICULE DE BREVET EUROPEEN

(45) Date de publication du fascicule du brevet:
**14.09.88**

(51) Int. Cl.⁴: **G 06 F 15/02, A 61 B 10/00**

(21) Numéro de dépôt: **84900708.3**

(22) Date de dépôt: **17.02.84**

(86) Numéro de dépôt international:
**PCT/CH 84/00025**

(87) Numéro de publication internationale:
**WO 84/03379 (30.08.84 Gazette 84/21)**

(54) **APPAREIL INDICATEUR DE L'ETAT ACTUEL DE FERTILITE D'UNE PERSONNE.**

(30) Priorité: **24.02.83 CH 1050/83**

(43) Date de publication de la demande:
**10.04.85 Bulletin 85/15**

(45) Mention de la délivrance du brevet:
**14.09.88 Bulletin 88/37**

(84) Etats contractants désignés:
**AT BE CH DE FR GB LI LU NL SE**

(56) Documents cités:
**EP - A - 0 022 060**
**EP - A - 0 031 251**
**US - A - 4 151 831**

(73) Titulaire: **Bioself International Inc., P.O.
Box 10496 Shirley Street 50, Nassau, Bahamas (BS)**
Titulaire: **Aeschlimann, Claude, 9, avenue Tronchet,
CH-1226 Thonex (CH)**

(72) Inventeur: **AESCHLIMANN, Claude, 9, avenue Tronchet,
CH-1226 Thonex (CH)**

(74) Mandataire: **Vuille, Roman et al, c/o KIRKER & Cie
S.A. 14, rue du Mont-Blanc Case Postale 872,
CH-1211 Genève 1 (CH)**

ACTORUM AG

## Description

On connaît déjà des appareils indicateurs de l'état actuel de fertilité d'une personne, au cours de son cycle d'ovulation et qui comprennent une source de tension, une sonde thermique destinée à être mise en contact avec le corps de la personne pour en mesurer la température, de préférence la température basale, un calculateur de fertilité et des moyens d'affichage de l'état de fertilité, commandés par ce calculateur (voir US-A-4 151 831, EP-A-0 022 060, EP-A-0 031 251).

Il est toutefois essentiel que les prises de température successives aient lieu à heure fixe, avec une marge de tolérance modérée, par exemple 24 h ± 2 heures. Les appareils connus auxquels il vient d'être fait allusion font confiance à la personne utilisant l'appareil et il peut parfaitement arriver que, dans la pratique, les prises de températures soient faites à des intervalles s'écartant, dans un sens ou dans l'autre, d'une marge excessive, ce qui fausse les calculs de l'appareil et, par conséquent, ses indications quant à l'état présent de fertilité de la personne. On sait en effet qu'il y a un rythme de variation journalier de la température chez chacun et que, par exemple, la température du corps d'un individu en bonne santé est toujours supérieure le soir à ce qu'elle est le matin. Des mesures de température à des intervalles irréguliers sont donc source d'indications trompeuses de l'appareil.

On a cherché à remédier à cet inconvénient (EP-A-0 031 251) en prévoyant qu'une mesure de température n'est acceptée, validée que si elle a lieu dans une «fenêtre temporelle» fixe de, par exemple, 4 heures. Ainsi, d'un jour quelconque au suivant, on est assuré que la mesure sera validée, c'est-à-dire prise en compte dans les calculs de l'appareil, si elle a lieu dans l'intervalle 24 ± 2 h., 24 heures étant l'intervalle de temps constant entre le milieu des fenêtres successives.

Toutefois, ce système de fenêtres fixes présente encore des inconvénients: rigidité pour l'usager, qui doit suivre un horaire invariable, possibilité d'un écart de temps malgré tout très important entre deux prises successives de température. En effet, si la fenêtre est de 4 h. et qu'un jour on prenne la température juste au début de la fenêtre et le lendemain juste à la fin de cette fenêtre, l'écart sera maximum et de 8 heures, ce qui a de sérieux inconvénients en ce qui concerne la fiabilité de l'appareil.

La présente invention vise à remédier à tous ces inconvénients en prévoyant une fenêtre temporelle fixe dont le début est automatiquement ajustable en fonction de l'instant de la précédente prise de température. Elle a pour objet un appareil qui est conforme à la revendication 1.

La figure 1 du dessin annexé est un schéma-bloc d'une forme d'exécution de l'appareil selon l'invention, donnée à titre d'exemple.

La figure 2 est un diagramme explicatif relatif à un affinement du travail de la forme d'exécution selon fig. 1.

La figure 3 est un schéma-bloc partiel destiné à compléter le schéma selon fig. 1 pour réaliser l'affinement selon fig. 2.

L'appareil comprend un élément thermosensible 1 incorporé dans une sonde thermométrique telle que l'une de celles décrites dans la publication WO 84/03378. A cet élément 1 est associé un détecteur de contact 2 qui peut être de l'un des types décrits dans le brevet précédent. Ce détecteur de contact peut se trouver sur la sonde thermométrique elle-même ou être constitué par un circuit particulier associé à l'élément thermosensible mais incorporé à l'intérieur de la sonde. A l'élément détecteur de contact 2 est associé un contrôleur de contact 3 dont la fonction est de vérifier constamment à partir du moment où le détecteur 2 a constaté que la sonde est en contact avec le corps de la personne dont on désire mesurer la température, que ce contact est maintenu ininterrompu pendant un certain laps de temps fixé d'avance. Ce laps de temps est prévu suffisant pour permettre à l'élément thermosensible 1 d'atteindre la température du corps, étant donné l'inertie thermique de la sonde et de l'élément thermosensible. Des formes d'exécution d'un tel contrôleur de contact ininterrompu sont également décrites dans le brevet précédent. En 4 se trouve un calculateur thermométrique de type connu qui convertit en valeurs numériques les valeurs de températures mesurées sous forme analogique par l'élément thermosensible 1. Ce calculateur 4 est prévu pour transférer les valeurs numériques qu'il calcule à un registre à décalage 5, par l'intermédiaire d'une porte 6 qui conditionne donc le transfert. 7 est un calculateur de fertilité dont la fonction est de déterminer sur la base de températures du corps relevées régulièrement toutes les 24 heures, l'état de fertilité d'une personne c'est-à-dire si la probabilité de conception est faible, élevée ou nulle. Il est connu qu'en effet c'est ainsi que les choses évoluent au cours de chaque cycle d'ovulation. Une forme d'exécution particulière de ce calculateur 7 est décrite dans la publication WO 84/03381.

Le calculateur de fertilité 7 est prévu pour agir sur un dispositif d'affichage comprenant, par exemple, trois lampes 8, 9, 10 destinées à indiquer respectivement que la probabilité de conception est faible, élevée ou nulle. Une porte 11 conditionne le fonctionnement effectif des lampes 8, 9 et 10 afin de limiter le temps de fonctionnement de ces lampes aux instants où l'on désire connaître l'état de fertilité, ceci afin de minimiser la consommation d'énergie de l'appareil. La commande de la porte 11 a lieu lors de l'entrée en fonction du détecteur de contact 2, par l'intermédiaire d'un timer 32 qui limite la durée d'ouverture de la porte 11.

L'appareil comprend une source de tension 12 et incorporée à l'appareil pour son alimentation.

Une référence de tension 13 est également prévue. Elle comprend, par exemple, une diode Zener assurant le maintien de cette tension de référence à partir de la source 12 elle-même. Un comparateur de tension 14 est prévu pour déterminer si la tension de la source 12 est acceptable ou si elle est tombée au-dessous du seuil d'utilisation. Si la tension est acceptable, 14 autorise la continuation du fonctionnement de l'appareil. Si, par contre, la tension est trop faible et par conséquent le fonctionnement de l'appareil pourrait être perturbé, le comparateur 14 ne donne pas le signal de continuation à la porte ET 15, si bien que la porte 6 ne peut en tout cas pas s'ouvrir et empêche le transfert de la température. Si, par contre, le comparateur 14 signale que la tension de la source est bonne et qu'en même temps le contrôleur de contact ininterrompu 3 signale qu'effectivement le contact a été ininterrompu pendant la durée fixée, la porte ET 15 laisse passer un signal allant à la porte ET 16. Si, au contraire, le comparateur de tension 14 donne un signal indiquant que la source de tension est hors d'usage, alors ce signal enclenche un avertisseur acoustique 17 par l'intermédiaire d'un inverseur logique 18 et d'une porte ET 33 si, simultanément, le timer 32 est en fonction.

L'appareil comprend une base de temps à quartz 19 émettant continuellement des impulsions, par exemple toutes les secondes. Un compteur 20 est commandé par la base de temps 19 et compte le temps de 0 à 22 heures au moyen des impulsions délivrées par 19. Lorsque le compteur 20 a totalisé 22 heures, il agit sur une porte ET 21 qui est reliée par ailleurs en 22 à la sortie de 19. Dès lors, les impulsions données par 19 passent par 21 et arrivent à un autre compteur 23 qui commence à totaliser les impulsions pendant une durée maximum de 4 heures.

Au moment où le compteur 20 est arrivé à 22 heures, d'une part il agit sur un inverseur 24, ce qui empêche la remise à zéro du compteur 23 jusqu'à nouvel avis. D'autre part, il vient agir sur la porte 16. Ainsi, si la porte 15 a laissé passer le signal et que le compteur 20 a envoyé le sien à la porte 16, celle-ci permet l'action d'ouverture sur la porte 6. En effet, à ce moment on sait, d'une part par le contrôleur 3 que le contact de la sonde a été ininterrompu pendant le temps voulu et, d'autre part, que la mesure est demandée pendant l'intervale de temps qui lui est accordé (24 heures $\pm$ 2 heures). Dès lors, la prise de température peut être faite, c'est-à-dire que le transfert de la calculatrice 3 au registre à décalage 5 peut avoir lieu et qu'en conséquence le calculateur de fertilité 7 peut fonctionner sur la base de cette nouvelle information. Si la porte 16 a permis l'ouverture de la porte 6, du même coup elle permet le passage du signal à travers la porte OU 25, ce qui provoque le reset du compteur 20. Lorsque le compteur 20 a été remis à zéro, sa sortie passe de 1 à zéro et de ce fait le reset du compteur 23 fonctionne par l'intermédiaire de l'inverseur 24 et remet ce compteur à zéro.

Si aucune prise de température n'a été effectuée durant les 4 heures de comptage du compteur 23, celui-ci envoie un signal par 26 à la porte 25 pour provoquer le reset du compteur 20. Ce cas correspond à l'absence de prise de température pendant la période de 4 heures autorisée par le compteur 23. Le signal envoyé par 26 agit simultanément sur le registre à décalage 5 par l'intermédiaire d'une porte OU 27, pour tenir compte du fait qu'un jour s'est écoulé sans prise de température.

Cette porte OU 27 permettant d'agir sur le registre 5, laisse également passer un signal chaque fois que la porte 6 est ouverte par la porte 16. Un appareil de signalisation acoustique 28 entre en activité lorsque le détecteur de contact a détecté le contact, mais que le compteur 23 indique qu'on est en dehors du temps prévu pour permettre cette mesure. Cela se produit de la façon suivante: le détecteur de contact 2 agit par 29 sur la porte ET 30, mais simultanément le compteur 20 qui a été remis à zéro parce qu'on a dépassé le délai de 4 heures en 23, donne par sa sortie un signal qui vient agir sur la porte 30 par l'intermédiaire d'un inverseur 31. Ce signal arrivant en même temps que celui que l'on a indiqué comme arrivant en 29, provoque le déclenchement du signal en 28 et l'usager est ainsi averti que la prise de température n'est pas acceptée.

Le fonctionnement de l'appareil décrit est le suivant.

Lorsque l'usager met la sonde en contact avec son corps, le détecteur 2 entre en fonction, ce qui déclenche le fonctionnement du contrôleur de contact ininterrompu 3, met en fonction le timer 32, ouvre la porte 11 et provoque l'affichage en 8, en 9 ou en 10, de l'état actuel de fertilité. Simultanément par 34, le timer 32 vient agir sur la porte 33, si bien que si la comparaison de tension en 14 indique que la source est défectueuse, le signal en 17 se produit. Si, au contraire, la source est en bon état, la porte 33 empêche le signal acoustique de 17 de se produire. Le détecteur de contact étant maintenu en activité parce que la sonde est ellemême maintenue en contact avec le corps, les opérations continuent ainsi: le contrôleur de contact ininterrompu 3 fonctionne et, si le compteur 23 indique qu'à ce moment on est dans la période de temps autorisant la prise de température, la porte 6 est ouverte par la porte 16. La porte 16 s'ouvre en effet parce qu'à ce moment-là le compteur 20 a atteint 22 heures et n'a pas été remis à zéro, parce qu'on est dans le délai de 4 heures indiqué par 23 et la valeur numérique de température calculée par 4 est transmise au registre à décalage 5. Ce registre fournit les indications au calculateur de fertilité 7. Ce registre établit le nouvel état de fertilité qui peut être identique au précédent ou différent de celui-ci, ceci pour autant que cette porte fonctionne. Le calculateur de fertilité peut dès lors obtenir du registre à décalage la valeur dont il a besoin pour effectuer son calcul de fertilité.

Si on est en dehors du délai de 4 heures fixé par le compteur 23, la prise de température est refusée par le signal fourni alors par le compteur 20 et agissant sur la porte 30 par l'intermédiaire de l'inverseur 31, le signal venant du détecteur 2 arrivant par ailleurs par 29.

L'opérateur a la possibilité d'arrêter ces opérations lorsqu'il le veut, simplement en faisant cesser le contact de la sonde avec son corps, ce qui provoque une réaction du détecteur de contact qui, à son tour, va agir sur le contrôleur de contact ininterrompu, d'une façon qui l'empêche d'agir sur la porte 15. Dès lors, les températures ne sont plus transmises de 4 en 5 parce que la porte 6 reste fermée. L'usager a ainsi la possibilité, s'il veut simplement connaître l'état actuel de fertilité, de provoquer l'affichage en 8, 9, ou 10 en mettant momentanément la sonde en contact avec son corps et en interrompant ce contact sitôt après qu'il a pris connaissance de l'état qui l'intéresse. L'appareil se remet ensuite automatiquement au repos.

Dans une variante, on pourrait se dispenser de la comparaison de tension en 14 et on pourrait provoquer l'affichage simplement en agissant sur un contact extérieur sans faire intervenir le détecteur de contact.

Il peut être avantageux d'avoir un signal acoustique ou lumineux indiquant la fin de prise de température. Ce signal pourrait tout simplement être commandé par le signal venant de la porte 16 et agissant sur la porte 6.

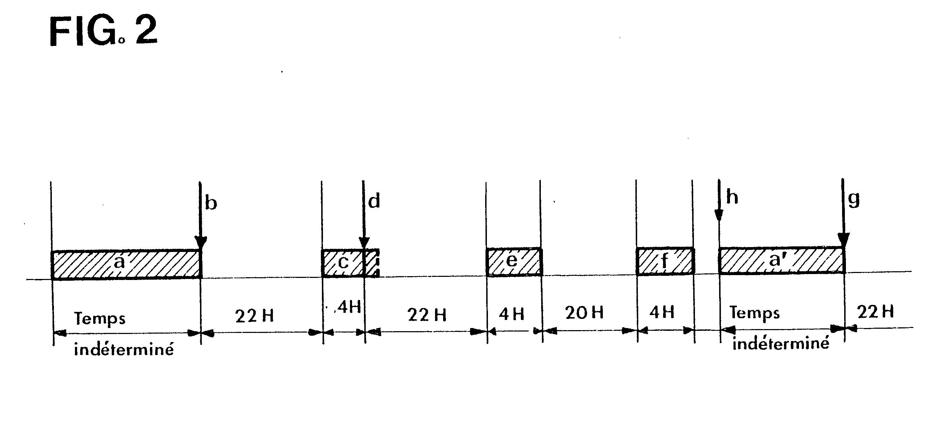

La fig. 2 illustre deux situations particulières, d'une part le cas de la mise en train de l'appareil suivie de l'omission d'une prise de température. D'autre part, ce diagramme illustre le cas où en cours d'utilisation on change de faisceau horaire et où il est désiré de pouvoir, dès ce moment, faire une prise de température à une heure qui correspond par exemple au début de la matinée dans le nouveau fuseau horaire.

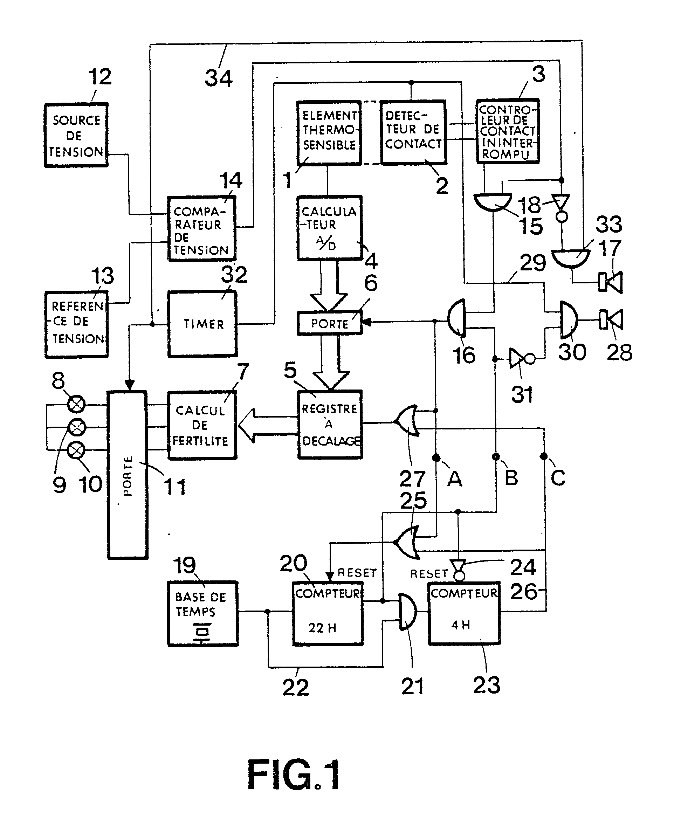

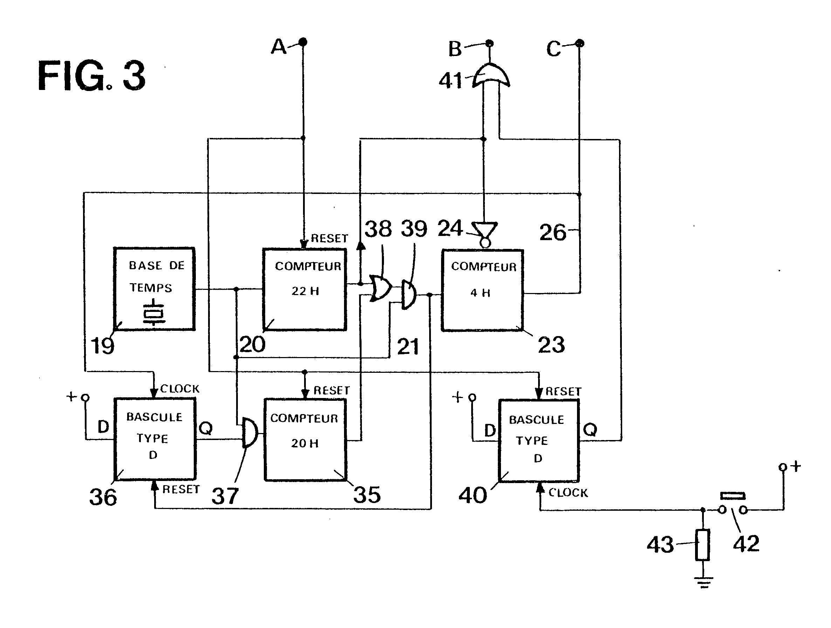

La fig. 3 est un schéma partiel représentant de quelle façon le circuit selon fig. 1 doit être modifié pour que l'appareil puisse traiter automatiquement les deux cas dont on vient de parler. On comprendra le circuit selon fig. 3 en admettant que les points indiqués A, B, C sont ceux auxquels il vient se raccorder aux points désignés de même façon sur la fig. 1 pour remplacer tout ce qui se trouve sur cette figure 1 au-dessous des points A, B, C.

Considérons d'abord le diagramme selon fig. 2. Il montre simplement en abscisses l'écoulement du temps à partir d'une période de longueur indéterminée au cours de laquelle l'appareil n'a pas encore été utilisé, soit a cette durée indéterminée. A la fin de cette première période a, l'usager procède à une première prise de température en b. Dès ce moment, le compteur 20 de 22 heures doit commencer à fonctionner, c'est-à-dire à totaliser des temps sous la commande de la base de temps 19. Au bout de la période de 22 heures, comme il a été expliqué précédemment, le compteur 23 de 4 heures est mis en route. Durant cette période de 4 heures indiquée en c, une deuxième prise de température a lieu en d. Dès ce moment, le compteur de 22 heures (20) a été remis à zéro et recommence à compter 22 heures. Si, dans la période e de 4 heures qui fait suite à cette deuxième période de 22 heures, la prise de température a lieu normalement, tout continue à se répéter comme il vient d'être décrit. Si au contraire, pour une raison quelconque, l'usager omet de faire une prise de température pendant la période e de 4 heures, alors l'appareil doit faire une correction en remplaçant la période suivante qui normalement serait de 22 heures par une période de 20 heures. La raison en est que si on ne faisait pas cette correction, la périodicité de 24 heures des mesures ne serait plus garantie mais se trouverait portée à 26 heures pour la première prise suivante et s'il y a plusieurs omissions de prise de température, on aurait ainsi donc une cumulation qui serait en contradiction avec la condition que les prises de température soit faites régulièrement toutes les 24 heures avec une marge toujours la même. Sur le diagramme, on a admis qu'après la période de 20 heures, il se produit une nouvelle période de 4 heures f, au cours de laquelle on n'effectue pas non plus de prise. Si tel est le cas, c'est une période de 20 heures qui devra suivre. Si au contraire une prise de température a lieu, ce sera une période de 22 heures qui suivra. Ce qui vient d'être indiqué correspond au premier cas.

Si on continue sur le diagramme, on admet qu'à un moment donné on désire changer l'heure de la prise de température, par exemple parce que l'usager est un voyageur qui a changé de fuseau horaire et qui va rester pendant un certain temps dans le nouvel horaire. Alors l'usager doit faire une manœuvre qui ramène l'appareil dans les conditions initiales, c'est-à-dire au début de la période a que l'on a indiquée ici en a'. Ayant fait cette manœuvre de remise au départ au moyen du circuit décrit plus loin, pendant un temps indéterminé l'appareil est prêt à effectuer une prise de température, en g, et à partir de ce moment les opérations recommenceront comme elles se sont déroulées depuis b. On a indiqué en h le moment où on a donné l'ordre de changement de fuseau horaire.

Voyons maintenant les moyens représentés sur le schéma fig. 3 prévus pour effectuer les fonctions que l'on vient de décrire.

Sur le schéma fig. 3, on voit en plus de la base de temps 19, du compteur 20 et du compteur 23, ainsi que des portes 21 et 24, que les éléments suivants sont ajoutés: un compteur 35 comptant des temps jusqu'à 20 heures sous la commande de la base de temps 19, une bascule 36 de type D commandant le compteur 35 par l'intermédiaire d'une porte ET 37. Le compteur 23 de 4 heures agit sur la bascule 36 lorsque le comptage de ce compteur 23 a dépassé 4 heures sans qu'une prise de température ait eu lieu. La bascule étant enclenchée, la porte ET permet le passage des impulsions de temps de la base 19 qui viennent ainsi mettre en route le compteur 35 de 20 heures. Les compteurs 20 et 35 agissent sur une porte OU 38 qui agit, à son tour, sur une porte ET 39. Cette porte commande le compteur de 4 heures 23 lorsque les deux compteurs 20 et 35 fonctionnent simultanément. La continuation du fonctionnement du compteur 20 n'a pas d'importance si le compteur 35 est en fonctionnement, car celui-ci arrive au bout de ce comptage avant l'autre. Ainsi donc, si le compteur 35 est en marche, il déterminera conjointement avec la base de temps 19 l'envoi d'impulsions de comptage dans le compteur 23 de 4 heures, ce qui permettra de réaliser la condition indiquée en f sur la fig. 2.

Ce qui vient d'être décrit jusqu'ici en référence à la fig. 3 concerne le premier cas traité à propos de la fig. 2, c'est-à-dire celui où une ou plusieurs prises de température ont été omises. Evidemment, dans le cas où aucune prise de température n'a été omise, le schéma selon fig. 3 ne change rien au fonctionnement décrit à propos de la fig. 1. Pour tenir compte de l'autre cas mentionné à propos de la fig. 2, c'est-à-dire celui du changement de fuseau horaire, il est prévu une bascule 40 de type D qui est remise à zéro chaque fois que la porte 6 (fig. 1) est ouverte par un signal venant de la porte 16.

Ce même signal remet aussi à zéro le compteur 20 qui dès lors va recommencer à compter jusqu'à 22 heures puis ensuite, comme décrit précédemment, lorsqu'on aura atteint 22 heures, c'est le compteur 23 qui va commencer à compter jusqu'à 4 heures et on se trouvera dans les conditions du diagramme fig. 2.

Cette bascule 40 agit sur une porte OU 41 sur laquelle agit aussi la sortie du compteur 20 lorsque l'on a atteint un comptage de 22 heures. La porte 41 laisse donc passer le signal en b destiné à la porte 6, lorsque les 22 heures sont dépassées ou que la bascule 40 est active. Il est aussi prévu un interrupteur 42 sur lequel l'usager vient agir pendant un bref instant pour faire repartir les opérations dans le cadre d'un nouveau fuseau horaire. En 43 se trouve une résistance de polarisation. La fermeture momentanée de l'interrupteur 42 a pour effet de remettre à l'état 1 la bascule 40 et, par conséquent, de laisser passer le signal allant à la porte 41. Dès lors, la prise de température peut avoir lieu et l'on se trouve dans les conditions g de la fig. 2. Ensuite les compteurs 22 et 23 vont assurer que les prises de température ultérieures auront lieu régulièrement toutes les 24 heures (avec une marge de tolérance) à partir de cette prise g.

Pour être complet, il convient d'ajouter qu'à l'origine du diagramme fig. 2 l'appareil n'était pas utilisé et était dépourvu de source de tension. C'est l'introduction de la source de tension qui a initié le fonctionnement, c'est-à-dire la période a d'attente qui a pris fin avec la première prise de température en b. Il va de soi que des périodes telles que a' correspondant à un changement de fuseau horaire peuvent avoir lieu autant de fois qu'on le veut. Il s'agit simplement, chaque fois, d'agir sur le bouton 42. La manœuvre de ce bouton a donc un caractère exceptionnel puisqu'elle n'intervient qu'en cas de changement de fuseau horaire.

L'interrupteur 42 et la résistance 43 pourraient être supprimés. Dans ce cas, ce serait l'enlèvement momentané de la source de tension 12 qui permettrait le changement de fuseau horaire. On se retrouverait ainsi en a. Des batteries tampons non représentées permettent évidemment de garder le contenu des mémoires, comme il est connu.

Les circuits logiques représentés dans les fig. 1 et 3 sont à logique câblée. Ils pourraient sans autre être remplacés par des circuits à logique programmée fonctionnellement équivalents utilisant un microprocesseur.

**Revendications**

1. Appareil indicateur de l'état actuel de fertilité d'une personne, au cours de son cycle d'ovulation, comprenant une source de tension (12), une sonde thermique (1), un calculateur (4) convertisseur en signaux électriques numériques de signaux électriques, fournis sous forme analogique par ladite sonde (1) suite à la mise en contact de la sonde avec le corps de la personne, un calculateur de fertilité (7) commandé par ledit calculateur convertisseur (4), pour fournir des signaux électriques indiquant l'état de fertilité de la personne, un récepteur de ces signaux (8, 9, 10), un contrôleur de temps (20, 23) pour n'autoriser qu'une prise de température par jour et pour ne la valider que si elle a lieu à l'intérieur d'une fenêtre temporelle journalière déterminée (de par ex. 4 heures), caractérisé en ce qu'il comporte des moyens (19–23) pour fixer chaque jour le début d'une fenêtre un premier nombre $N_1$ d'heures après la prise précédente – $N_1$ étant 24 heures moins $N_{2/2}$ et $N_2$ la durée d'une fenêtre –, assurant ainsi automatiquement l'adaptation de la position de la fenêtre en cas de changement de l'heure de mesure, à l'intérieur de cette fenêtre.

2. Appareil selon la revendication 1, caractérisé en ce que les moyens pour fixer, chaque jour, la position de la fenêtre temporelle de durée déterminée sont agencés pour fixer le début de la fenêtre du jour suivant celui de la mesure à 24 heures moins la moitié de la durée de la fenêtre.

3. Appareil selon la revendication 1 et comportant un contrôleur (13, 14) d'état de la source de tension (12), un détecteur de contact (2) de la sonde (1) avec le corps de la personne, pour initier le fonctionnement du contrôleur de temps (20, 23), et un contrôleur (3) de contact ininterrompu de la sonde (1) avec le corps, pour autoriser la prise de température seulement si le contact de la sonde avec le corps est ininterrompu pendant un laps de temps fixé d'avance et au moins égal au temps nécessaire à la sonde, en raison de son inertie thermique, pour atteindre la température à mesurer, caractérisé en ce que le détecteur de contact (2) est agencé pour déclencher le fonctionnement du contrôleur (13, 14) d'état de la source de tension (12), du contrôleur de contact ininterrompu (3), et de moyens d'affichage (8, 9, 10) de l'état de fertilité, et en ce que le contrôleur de temps (20, 23) est agencé pour conditionner la prise de température et le fonctionnement du calculateur de fertilité (17), l'arrêt des opérations étant commandé par le détecteur de contact (2) lorsque la personne fait cesser le contact de la sonde (1) avec son corps, pour remettre l'appareil au repos.

4. Appareil selon la revendication 1 ou 2, caractérisé en ce qu'il comporte des moyens assurant qu'en cas d'omission d'au moins une prise de température journalière, le nombre $N_1$ d'heures est réduit à une valeur $N_1'$ telle que $N_1' + N_2 = 24$, le comptage de $N_1$ démarrant à l'issue de la fenêtre au cours de laquelle il n'y a pas eu de mesure.

5. Appareil selon la revendication 1 ou 2, caractérisé en ce qu'il est agencé pour que, si la source de tension est rendue inopérante pendant un court laps de temps (par ex. 90 secondes), d'une part les données mémorisées sont conservées et, d'autre part, la prochaine mesure de température constitue un nouveau départ, indépendant de la mesure précédente, pour la fixation automatique de la fenêtre temporelle du jour suivant, pour permettre à l'usager de s'adapter à un changement de fuseau horaire.

## Claims

1. An apparatus for indicating the current state of fertility of a person during her ovulation cycle, comprising a voltage supply (12), a temperature probe (1), a unit (4) for converting, to digital electrical signals, electrical signals produced in analogue form by the said probe (1) after the probe has been brought into contact with the person's body, a fertility calculator (7), controlled by the said converter (4), for producing electrical signals indicating the state of fertility of the person, a unit (8, 9, 10) for receiving these signals and a time monitor (20, 23) for allowing the temperature to be measured only once a day and for validating this measurement only if it takes place within a given daily time window (of e.g. 4 hours), characterized in that it comprises means (19–23) for fixing, each day, the beginning of a window a first number of hours $N_1$ after the previous measurement – $N_1$ being 24 hours minus $N_2/2$ and $N_2$ being the duration of a window – thereby automatically ensuring that the position of the window is appropriate in the event of a change in the time of measurement, within this window.

2. The apparatus according to Claim 1, characterized in that the means for fixing, each day, the position of the time window of given duration are arranged so as to fix the beginning of the window on the day following that of measurement at 24 hours minus half the duration of the window.

3. The apparatus according to Claim 1, comprising a unit (13, 14) for monitoring the state of the voltage supply (12), a unit (2) for detecting contact between the probe (1) and the person's body so as to initiate the operation of the time monitor (20, 23), and a unit (3) for monitoring uninterrupted contact between the probe (1) and the body so as to allow the temperature to be measured only if contact between the probe and the body is uninterrupted for a preset period of time at least equal to the time required by the probe, because of its thermal inertia, to reach the temperature to be measured, characterized in that the contact detector (2) is arranged so as to trigger the operation of the unit (13, 14) for monitoring the state of the voltage supply (12), the uninterrupted contact monitor (3) and means (8, 9, 10) for displaying the state of fertility, and in that the time monitor (20, 23) is arranged so as to condition the temperature measurement and the operation of the fertility calculator (17), the cessation of the operations being actuated by the contact detector (2) when the person breaks contact between the probe (1) and her body in order to bring the apparatus out of use.

4. The apparatus according to Claim 1 or 2, characterized in that it comprises means for ensuring that, in the event of at least one daily temperature measurement being omitted, the number of hours $N_1$ is reduced to a value $N_1'$ such that $N_1' + N_2 = 24$, the counting of $N_1$ starting at the end of the window during which no measurement has taken place.

5. The apparatus according to Claim 1 or 2, characterized in that it is arranged so that, if the voltage supply is rendered inoperative for a short period of time (e.g. 90 seconds), on the one hand the stored data are preserved and on the other hand the next temperature measurement represents a fresh beginning, independent of the previous measurement, for automatic fixing of the time window on the next day, so as to enable the user to adapt to a change in time zone.

## Patentansprüche

1. Gerät zur Angabe des aktuellen Fruchtbarkeitszustandes einer Person während ihres Ovulationszyklus, bestehend aus einer Spannungsquelle (12), einer thermischen Sonde (1), einem Rechner (4) zur Umwandlung in elektrische Digitalsignale von elektrischen Signalen, die von besagter Sonde (1) geliefert werde bei Kontakt der Sonde mit dem Körper der Person, einem Fruchtbarkeitsrechner (7), der von besagtem Umwandlungsrechner (4) gesteuert wird, um elektrische Signale zu liefern zur Angabe des Fruchtbarkeitszustandes der Person einem Empfänger für diese Signale (8, 9, 10), einem Zeitüberwacher (20, 23), der nur eine einzige Temperaturmessung pro Tag zulässt und diese Temperaturmessung nur dann als gültig werten lässt, wenn sie innerhalb eines bestimmten täglichen Zeitfensters (zum Beispiel innerhalb von 4 Stunden liegt), dadurch gekennzeichnet, dass es Mittel (19–23) enthält, um jeden Tag den Anfang eines Fensters einer ersten Anzahl $N_1$ Stunden nach besagter Messung festzulegen – wobei $N_1$ gleich 24 Stunden minus $N_2/2$ ist und $N_2$ die Dauer eines Fensters ist – wodurch die Lage des Fensters, im Falle einer Änderung der Messstunde im Innern dieses Fensters, automatisch angepasst wird.

2. Gerät nach Patentanspruch 1, dadurch gekennzeichnet, dass die Mittel zur täglichen Festlegung der zeitlichen Lage des Fensters bestimmter Dauer derart ausgebildet sind, dass sie den Anfang des täglichen Fensters festlegen gemäss jenem der 24-Stunden-Messung minus der Hälfte der Dauer des Fensters.

3. Gerät nach Patentanspruch 1, und enthaltend einen Überwacher (13, 14), um den Zustand der Spannungsquelle (12) zur überwachen, einen Detektor (2) der Sonde (1), um die Kontaktgabe mit dem Körper der Person festzustellen und den Zeitüberwacher (20, 23) in Gang zu setzen, und einen Überwacher (3), um die ununterbrochene Kontaktgabe der Sonde (1) mit dem Körper zu überwachen zur Freigabe der Temperaturmessung nur dann, wenn die Kontaktgabe der Sonde mit dem Körper ununterbrochen während eines zum voraus festgelegten Zeitraumes erfolgt und mindestens gleich der Zeit ist, die aus Gründen der thermischen Trägheit der Sonde erforderlich ist, um ihre Messtemperatur zu erreichen, dadurch gekennzeichnet, dass der Kontaktdetektor (2) so aufgebaut ist, dass er den Überwacher (13, 14) des Zustandes der Spannungsquelle (12) und den Überwacher für ununterbrochene Kontaktgabe (3) freigibt, und Anzeigemittel (8, 9, 10) für den Fruchtbarkeitszustand und dass der Zeitüberwacher (20, 23) so aufgebaut ist, dass er die Temperaturmessung und den Beginn des Funktionierens des Fruchtbarkeitsrechners (17) auslöst, wobei das Abstellen aller Funktionsabläufe durch den Kontaktdetektor (2) gesteuert wird, wenn die Person die Sonde (1) ausser Kontakt mit ihrem Körper bringt, um das Gerät in den Ruhezustand zurückzuversetzen.

4. Gerät nach Patentanspruch 1, oder 2, dadurch gekennzeichnet, dass darin Mittel enthalten sind, die bei Auslassen mindestens einer der täglichen Temperaturmessungen bewirken, dass die Stundenanzahl $N_1$ auf einen solchen Wert $N_1'$ herabgesetzt wird, dass $N_1' + N_2 = 24$ ist, wobei die Zählung von $N_1$ gestartet wird am Ende des laufenden Fensters während dem keine Messung durchgeführt wurde.

5. Gerät nach Patentanspruch 1 oder 2, dadurch gekennzeichnet, dass es so aufgebaut ist, dass, wenn die Spannungsquelle während eines kurzen Zeitraumes (zum Beispiel während 90 Sekunden) inaktiviert wird, einerseits die gespeicherten Daten nicht verloren gehen und, andererseits, die nächstfolgende Messung einen neuen Start bewirkt, und zwar unabhängig von der vorhergehenden Messung, zur automatischen Festlegung des Zeitfensters des folgenden Tages, um dem Benutzer zu ermöglichen sich an einen Wechsel der Zeitzone anzupassen.

**FIG.1**

FIG. 2

FIG. 3